Europäisches Patentamt

European Patent Office

Office européen des brevets

(11) Publication number: 0 166 567
A2

# (12) EUROPEAN PATENT APPLICATION

(21) Application number: 85304356.0

(22) Date of filing: 18.06.85

(51) Int. Cl.⁴: **G 01 N 23/04**, H 05 G 1/64,
G 01 T 1/29, G 21 K 1/02

(30) Priority: 21.06.84 US 622888

(43) Date of publication of application: 02.01.86
Bulletin 86/1

(84) Designated Contracting States: DE FR GB NL

(71) Applicant: PICKER INTERNATIONAL, INC., 595 Miner
Road, Highland Heights Ohio 44143 (US)

(72) Inventor: Mattson, Rodney A., 6532 Melshore Drive,
Mentor Ohio 44060 (US)
Inventor: DeMeester, Gordon D., 1613 Dennis Drive,
Wickliffe Ohio 44092 (US)
Inventor: Tesic, Michael M., 17838 Landseer Road,
Cleveland Ohio 44119 (US)

(74) Representative: Pope, Michael Bertram Wingate, Central
Patent Department Wembley Office The General Electric
Company, p.l.c. Hirst Research Centre East Lane,
Wembley Middlesex HA9 7PP (GB)

(54) Imaging system and method.

(57) An imaging system includes an x-ray source, and means
for scanning x-rays from the source through a subject and
across the input portion (313 or 360) of an area detector (312
or 353) covering at one time only a fraction of the area detec-
tor's input. The area detector produces at its output portion a
moving representation of the x-rays being scanned across the
input. Means are provided for continuously projecting the mov-
ing representation onto a fixed portion of a second stationary
discrete solid state detector.

PKI/2652 EPC

Imaging System and Method.

This invention relates generally to the field of diagnostic imaging, and more particularly to digital scan projection radiography, and systems and methods for improving frame repetition rates in such imaging while retaining good dynamic range in imaging and high scatter rejection.

Radiographic systems and techniques have long been used in medical diagnostics. In a typical conventional radiographic system, a patient is positioned between an x-ray source and a radiographic screen which serves as a radiation detector. X-rays from the source pass through the patient's body and emerge in a pattern to fall incident upon the screen, which produces an image describing the pattern. The image provides radiologists with information relating to the internal structure or condition of the patient's body.

In conventional radiography, the screen comprises a layer of radiographic film sensitive to electromagnetic radiation in both visible and invisible portions of the spectrum. The layer of film is sandwiched between x-ray intensifier screens. The image is produced by processing the film after x-ray exposure.

Conventional radiographic systems, while simple and economical, and having good spatial and temporal resolution characteristics, have several disadvantages. Conventional radiographic systems have quite limited power to detect low contrast levels in an image. The display is rather inflexible. Storage and retrieval of images is expensive and cumbersome.

Also, conventional radiography can produce only a shadow image, in which portions of the body of no interest can sometimes obscure the body portions desired to be imaged.

Other types of diagnostic imaging systems and techniques are known as fluorography and fluoroscopy. Such systems employ a source for directing x-rays through a patient to be examined, to emerge therefrom in a pattern. The pattern is directed to an input face of an image intensifier tube. The image intensifier tube responds to an incident pattern of x-rays on its input face to produce, at an output face, a smaller relatively bright visible light image corresponding to the pattern of x-rays falling upon the input face. A video system including a high quality television camera views the output face and produces a television display representing internal structure of the patient's body as defined by the pattern of x-rays.

One disadvantage with such systems is that, even with state of the art television equipment, the video signals produced by the camera have a relatively limited dynamic range. An advantage of such systems, however, is their relatively fast frame repetition rate capability, which can if desired produce a virtually real time image.

More recently, digital radiographic systems have been proposed which produce image information whose quality and flexibility are in some ways superior to that produced in conventional radiographic and fluorographic imaging. Digital radiography systems are often categorized by the geometry of x-ray beams they employ. Systems considered most promising include so-called "area beam" systems and "scan projection radiography" (SPR) systems.

In a typical area beam digital radiographic system, a divergent cone of x-rays is directed through the patient's body toward the input face of an image intensifier tube.

A video camera views the intensifier tube output face and produces, as in conventional fluorography, video signals corresponding to the light image. These video signals are then digitized. In their digital form, the signals are susceptible to many types of useful digital processing, such as contrast enhancement. Additionally, the digital signals are susceptible of storage and retrieval in very convenient form, such as on tape or disc, and can be transmitted readily to remote locations.

Moreover, digital systems lend themselves well to techniques of temporal and energy subtraction, which if employed can reduce or eliminate the disadvantage of conventional shadow imaging by actually removing unwanted portions of the body from the image.

According to another proposal, area beam digital radiography can be practiced by the use of a solid state large area detector comprising a rectangular arrangement of discrete detector elements. A phosphor layer overlies the detector array and converts x-rays incident upon it to visible light. Each detector element responds to incident light to produce an analog electrical signal representing the incident energy which caused its production. The respective electrical signals collectively represent the pattern of x-rays emergent from the patient's body.

Circuitry coupled to each of the detector elements amplifies and digitizes these analog electrical signals. Digital processing circuitry and equipment responds to the digitized signals to produce a digital representation of a diagnostic image.

4

While digital radiography represents a significant advance in some respects, problems do exist. For instance, because the entire screen is simultaneously actuated to detect x-rays, some radiation scatter, which always occurs, is detected over the entire screen and in effect "blurs" the image. Scatter radiation falls in a rather diffuse fashion onto the detector. This causes the production of spurious signals not indicative of the image pattern of the primary radiation energy emergent from the patient's body.

Also, optical crosstalk can occur among neighboring discrete detector elements.

Another problem is that, since the entire large field is illuminated simultaneously, data is generated simultaneously representing the whole detected image. This high rate of data generation requires more expensive and sophisticated digital processing equipment than would be the case if data were produced more slowly.

Data transfer rates are limited by disc transfer rates and by availability of data compression hardware. More importantly, the sheer number of detector elements and information channels complicates implementation of prior art techniques.

Scan projection radiography eliminates or reduces some of the problems inherent in area beam radiography. Scan projection radiography (SPR) utilizes a smaller detector array than area beam radiography, and acquires data for large area imaging by synchronous scanning movement of the source and detector array relative to the patient.

In one proposed type of SPR, the x-ray beam from the source is collimated by a suitable slit defining structure into a thin spread beam lying substantially in a plane. The detector assembly comprises a generally elongated array of discrete detector elements. Each detector element can suitably comprise, for example, a photodiode with a phosphor layer optically coupled to a receiving light sensitive surface facing the radiation source.

Mechanism is provided for effecting relative movement between the source-detector arrangement and the patient. Such movement can take the form of pivoting the radiation source about an axis extending through the focal point of x-ray beam generation, combined with a corresponding synchronous movement of the detector, either along an arc of a circle or a line, such that all the detector elements remain, during this motion, aligned with the collimated spread beam which is directed through the patient.

In the course of this movement, the spread beam passes through a succession of planes of the patient's body. The detector simultaneously produces information relating to the pattern of spread beam radiation which emerges from the body and is seen by the detector as a transversely moving line. The spread beam illuminates simultaneously all the detector elements of the elongated detector array.

The detector output is repeatedly read out to generate a succession of signal sets. Each set represents radiation emergent from the patient's body along the line defined by the location of the spread beam at the instant of detector readout. Together, these individual "line scans" make up an entire image.

Scan projection radiography provides some advantage over area beam digital radiography, but at the expense of some disadvantages.

It is well known, for instance, that line scan projection radiographic systems can provide improved detection of low contrast subjects by reducing the ef-

fect of scatter radiation. This advantage results from the fact that both the spread beam of radiation and the detector array used in SPR are relatively thin and does not therefore cause or detect significant scatter radiation from regions of the patient's body outside the line being instantaneously examined. Thus, there is less opportunity than in area beam radiography for scatter to produce spurious signals.

Scan projection radiography does, however, have some significant disadvantages. The flux of radiation from the source which is available for detection by the detector array is limited by the thinness of the collimated spread beam and, of course, by the output from the x-ray source. With less radiation falling upon the detector elements, these elements can become "light starved" with a consequent degradation of contrast in the resulting image.

Another important disadvantage of scan projection radiography is that of its relatively low frame repetition rate capability. As noted above, in SPR imaging, the detector, any collimators, and the x-ray tube must all move in synchrony for imaging to occur. The considerable mechanical inertia of these elements, and the corresponding demands on the mechanical power system for moving them, prevents a frame repetition rate which is sufficiently high for some purposes.

It is an object of this invention to provide an imaging system and method which combines the relatively high frame repetition rate of area beam imaging systems with the superior scatter rejection and dynamic range of SPR systems and of discrete, solid state detector technology.

The disadvantages of the prior art are overcome or reduced by an x-ray imaging system comprising an x-ray source, an area detector having input and output portions, and means for scanning x-rays from the source through a subject and across the input portion of the area detector in a pattern covering at one time only a portion of the area detector's input. The area detector produces at its output portion a moving representation of the x-rays being scanned across the input.

The system also includes a second detector and means for projecting the moving representation of scanned x-rays continuously onto a fixed portion of the second detector. In the preferred embodiment, the second detector is mounted fixed with respect to the area detector. The second detector can comprise but a single row of detector elements.

The imaging system further comprises data transmission circuitry coupled to the output of the second detector. The data transmission circuitry restores to this output, produced by a stationarily projected input to the second detector, information describing the x-ray scanning motion. This is achieved by removing output data from the second detector in a temporal sequence corresponding to the scanning motion.

This system combines the rapid frame rate capability of an area beam system with the superior scatter rejection of scanned projection radiography systems.

The second detector, as noted, can thus be stationary. This system thus eliminates the need in previous SPR systems for cumbersome and expensive gantry mechanism for scanning the detector and other components with respect to the subject to be imaged. The elimination of the need for consideration of the mechanical parameters of such a mechanical system dramatically increases the frame rate capability of this inventive system.

Also, a relatively small second detector can be used. The input portion of that detector need be only large enough to accommodate the continuous fixed presentation thereto of a relatively small area pattern of the scanned x-rays.

Also, this small second detector may be of the discrete array solid-state type. Such a detector provides a viable substitute for previously used video systems to detect the output of an area detector. Thus, the relatively limited dynamic range of such video systems need not be tolerated. Rather, the system can employ an output detector which is both economically practicable, because of its relatively small size, and able to employ a discrete solid state array, with its superior dynamic range.

According to a more specific embodiment, the second detector comprises parallel rows of individual detector elements defining a rectangular array. Time delay and integrate circuitry is coupled to respective rows of the individual detector elements. Clocking circuitry causes the time delay and integrate circuitry to shift and accumulate charge signals, produced by the detector elements, along the respective rows of individual detector elements. The shifting and accumulating is in a directional sense opposite to that of the scanning of the x-rays across the input portion of the area detector. Output circuitry removes the accumulated and shifted charge signals from the second detector as they reach end elements of the respective rows of detector elements.

In accordance with a more specific feature, the clocking circuitry shifts and accumulates the charge signals at a scalar average velocity corresponding to the average velocity of x-ray scanning across the area detector input.

More specifically, in the preferred embodiment, the width of the x-ray pattern scanned across the area detector is chosen such that the corresponding projection onto the second detector is equal in width to that of the second detector array. The average charge shifting velocity is chosen such that charge is shifted across the detector array width in the time required for the x-ray pattern to scan a distance equal to its own width.

The use of the time delay and integrate circuitry in combination with the rectangular matrix of individual detector elements substantially enhances the signal to noise ratio of the output signals from the second detector which are employed to produce the subject image.

More specific features of this system include the use of an image intensifier tube as an area detector and an x-ray source disposed for directing the x-rays through the subject and toward the input face of the intensifier tube. The scanning apparatus includes apparatus defining a slit interposed between the source and the image tube and mechanism for moving the slit sidewards and transversely to the path of the x-rays directed to the input face. This structure scans a moving slit of x-ray across the subject and intensifier tube input face. This slit scan of x-ray produces a corresponding slit light pattern which moves transversely across the output face of the image tube.

A novel optical system is provided for continuously projecting the moving slit light pattern appearing at the image tube output face onto a fixed portion of the second detector input. This optical system includes a pivotable mirror or prism positioned to intercept at least a portion of the light energy from the image tube output face. Synchronization apparatus and circuitry is coupled between the pivotable mirror and the x-ray scanning apparatus comprising the slit defining structure. The synchronization apparatus and circuitry synchronizes the transverse movement of the x-ray scanning apparatus with the pivoting motion of the pivotable mirror to continuously maintain the projection of the slit light pattern onto the same portion of the second detector, during movement of the slit light pattern across the image tube output face.

In accordance with another specific feature of the invention, the means for presenting the scanned x-ray pattern from the area detector output to the second detector comprises a beam splitter, which is employed to transmit continuously a portion of the area detector output energy to a known type of video system. In such a system, the means for scanning x-rays across the input face of the area detector can be eliminated altogether when desired. The system can thus be operated in a conventional video mode, with the full input face of the area detector illuminated with x-rays, and the video system viewing the entire output face.

In accordance with a more general aspect of the invention, an imaging system is provided utilizing a source for directing penetrative energy through different portions of a subject to form collectively a single area pattern of emergent energy. A detector responsive to such energy produces a sequence of differently spatially located representations of portions of the emergent energy pattern. The system comprises means for transmitting each such representation incident substantially upon the same fixed region of a stationary second detector, and for employing the second detector output to produce a representation of internal subject structure.

It is an object of this invention to provide an x-ray imaging system which combines the scatter rejection of SPR systems, the dynamic range of discrete solid state detectors and the relatively high frame rates of area detector systems.

0166567

These and other aspects of the present invention will be understood in more detail by reference to the following description, and to the drawings, in which:

Figure 1 is a pictorial drawing illustrating the major components of a digital scan projection radiography system;

Figure 2 is a generalized block diagram of the system of Figure 1;

Figure 3 is an elevational view of a portion of the system of Figure 1;

Figures 4A-4D are partly pictorial, partly block illustrations exemplifying in simplified form an operation sequence of the system of Figure 1;

Figure 5 is a detailed elevational view illustrating a portion of the system of Figure 1;

Figures 6 and 7 are side views illustrating embodiments of the portion of the system shown in Figure 5;

Figure 8 is a pictorial view of an alternate embodiment of a portion of the system of Figure 1;

Figures 9 and 10 are pictorial views illustrating geometrical component relationships and modes of operation of a portion of the system of Figure 1;

Figures 11 and 12 are detail top views illustrating a portion of the system shown in Figure 1;

Figure 13 is a detail block diagram illustrating a portion of circuitry of the system of Figure 1; and,

Figure 14 is a graphical timing diagram illustrating operation of a portion of the system of Figure 1.

Figures 15 and 16 are top views, partly pictorial and partly in block form, illustrating an alternate embodiment of an imaging system incorporating the presently claimed invention.

Figures 17-19 are detail drawings illustrating specific aspects of the system of Figure 15.

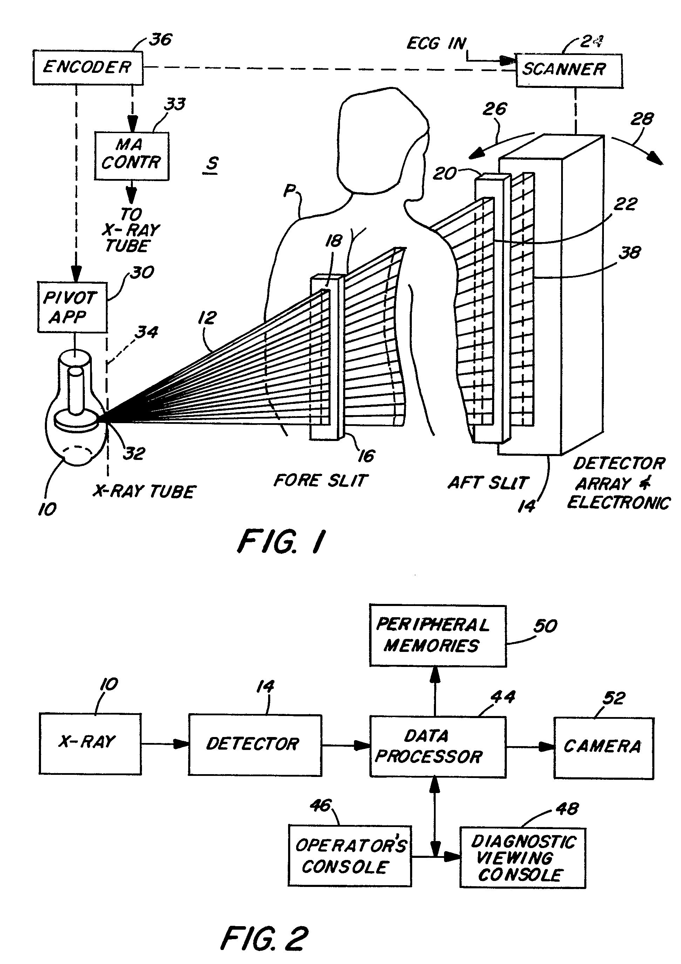

A system S for performing digital scan projection radiography (SPR) is illustrated in general form in Figure 1. The system S directs a pattern of x-rays through a patient P and produces, from information borne by the x-ray pattern emergent from the patient's body, a tangible representation, generally in the form of a visible image, describing the internal structure or condition of the patient's body.

The system S incorporates an x-ray source 10 for directing a beam of x-ray energy illustrated as a collection of rays 12 through the patient P and onto a multi-element detector assembly 14. A first collimator structure 16 defines a generally vertical fore slit 18 for collimating the x-rays emanating from the source into a spread beam lying generally within a vertical plane. A second collimator structure 20 defines an aft slit 22 located between the patient and the detector assembly for further enhancing this collimation. This feature contributes to the very important goal of scatter rejection.

Gantry structure (not shown) connects the collimators 18, 20 to the detector assembly 14 to maintain mutually constant alignment between the collimators and the detector. Alternately, servo linking, rather than mechanical linkage, is possible between the detector and collimators.

Mechanical scanner apparatus 24 is coupled to the detector assembly 14 to move the detector along an arcuate path defined by arrows 26, 28. The gantry structure maintains alignment between the collimator structures 16, 20 and the detector during detector motion.

Pivoting apparatus 30 is coupled to the x-ray source The apparatus 30 pivots the source synchronously with detector arcuate motion to continuously track the detector 14 and the mutually aligned collimators 16, 20.

The x-ray source 10 comprises an x-ray tube, and associated power circuitry (not shown) for electrically actuating the tube to produce the x-rays (in pulsed or continuous mode) emanating from a focal spot 32 defined by the structure of the tube. The pivoting motion effected by the pivot apparatus 30 causes the tube to pivot about a vertical axis 34 extending through the focal point 32.

It is believed preferable to couple the detector to the master drive of the scanner apparatus and to control the tube to follow, since detector positioning, and its synch with other circuitry as described below, is more critical than tube position.

An encoder 36 is coupled to the scanner apparatus 24 and produces a signal indicating the instantaneous position of the detector 14 along its arcuate path described by the arrows 26, 28. The output of the encoder 36 is directed to the pivot apparatus 30 for synchronizing the pivoting motion of the x-ray tube 10 with the arcuate motion of the detector 14 and collimators 16, 20, to maintain continuous alignment between the x-ray beam, collimators and detector assembly during scanning motion. The encoder also synchronizes the readout of accumulated signals from the respective detector elements.

The scanner apparatus can be appropriately synched with a physiological signal, such as ECG, or with a signal indicating timing of administration of a contrast agent (see Fig. 1). Temporal subtraction studies can also be done with sufficiently rapid retrace between scans, which, as explained below, is possible with this system.

An example of a type of encoder apparatus is described in U.S. Patent No. 4,015,129, issued on March 29, 1977 to Manring, et al, incorporated by reference.

The encoder 36 is also coupled to the m.a. (current) control 33 of the x-ray tube 10. The encoder adjusts tube m.a., and hence, the intensity of x-ray output, as a function of the location of the detector along its scanning path. In the embodiment described here, the tube m.a. is controlled to decrease as a function of the degree of detector displacement from the middle position along its scanning path. Thus, where the patient's body is less thick, i.e. near its right and left sides, x-ray output is reduced to maintain a more uniform x-ray flux at the detector throughout its scan.

The detector 14 includes an array of individual detector elements, generally arranged in the region of an elongated slot 38 of the detector assembly 14. The structure and arrangement of the detector elements is described in detail below. Each of the detector elements responds to light energy (generated by x-rays as described below incident on a receiving face thereof to produce an electrical signal which represents a characteristic of the x-ray which caused the production of the electrical signal.

In operation, the detector, collimators and x-ray tube are moved to the left as in the direction illustrated by the arrow 26 to prepare for a scan. In performing a scan, the x-ray tube 10 is actuated to produce x-ray energy. The scanner apparatus 24 and pivot apparatus 30 is operated to synchronously scan the vertical spread beam of x-rays from left-to-right in Figure 1 across the patient's body. During this scanning motion, the detector elements of the detector assembly 14 produce analog electrical signals. The electrical signals produced by the array of detector elements is rapidly and repeatedly sampled to produce sequential representations of image data corresponding to closely spaced vertical lines through the patient's body over the course of the scan.

There are two ways by which data sampling can be triggered. One is by triggering at each of a set of predetermined time increments. Another is to trigger by way of a position encoder as described above.

The electrical signals are then digitized and processed to produce the desired patient imaging.

Figure 2 illustrates a generalized block diagram of the system of Figure 1. In Figure 2, the x-ray source 10 directs x-rays to the detector assembly 14. Signals from the detector 14 are transmitted to a data processor 44 which digitizes and processes the electrical signals. In response to commands from an operator's console 46, the data processor 44 produces various types of tangible representations of internal body structure of the examined patient. In one mode, the data processor actuates a diagnostic viewing console 48 to produce directly a visible image of the patient's internal body structure which can be immediately employed by a radiologist for medical diagnostic purposes.

In another mode, the data processor 44 stores digital information representing patient image data in one or more peripheral memories 50. Optionally, a camera 52 can be coupled to the data processor.

Digital scan projection radiography systems are described in the following publications and their bibliography, all of which are specifically incorporated by reference: Mattson, R. A. et al "Design and Physical Characteristics of a Digital Chest Unit", S.P.I.E. Vol. 314, Digital Radiography (1981); Digital Radiography, the General Electric Company, 1980; Digital Fluorography, the General Electric Company, 1981.

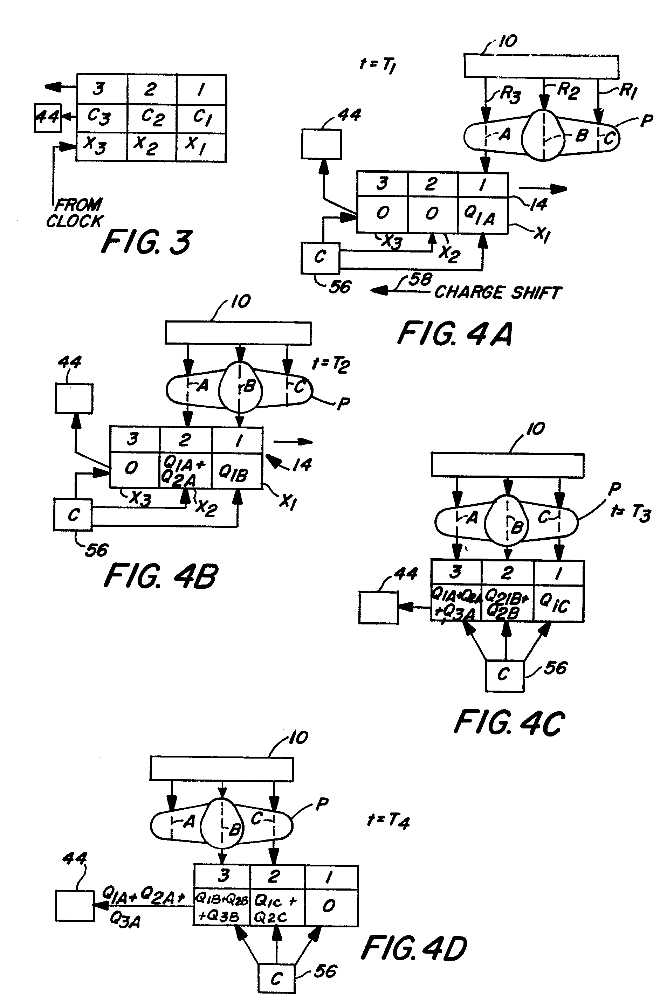

Operation of the detector assembly 14 embodying the present invention is believed initially best described by means of a highly simplified example. This example is illustrated mainly by reference to Figures 3 and 4A, 4B, 4C and 4D.

The operation of the detector assembly 14 is in accordance with a technique which is sometimes referred to as "time delay and integrate" (TDI). Figure 3 illustrates a greatly simplified form of detector element arrangement incorporated into the detector assembly 14. Figure 3 shows a row of three individual detector elements 3, 2, 1. The detector elements 3, 2, 1 are arranged in an adjacent linear configuration extending horizontally, with respect to Figure 1, across a portion of the slot 38 of the detector assembly 14. In practice, there are far more than three detector elements in each such row, and also there are a great number of horizontal rows of such detectors distributed vertically along the entire height of the slot 38. For purposes of explanation, however, only this single row of detectors will be initially considered.

Referring to Figure 4A, there is shown a simplified representation of an x-ray source 10. The x-ray source 10 emits x-radiation through the patient P toward the detector assembly 14. In this simplified diagram, the x-ray source 10 may be thought of as a source of parallel rays R1, R2, R3 of x-ray emitted simultaneously in a downward direction as shown in Figure 4A. The lateral dimension of the source 10 is greatly exaggerated, relative to the patient, for purposes of simplicity and clarity. In actuality, however, the lateral dimension of the x-rays directed to the patient is defined by the thickness of the spread beam which penetrates the patient after collimation by the collimator 16.

Each of the detector elements 3, 2, 1 has circuitry associated with it, including a storage capacitor (shown in Figure 4A in block form as C3, C2, C1), for storing

and maintaining an analog electrical charge signal. The charge is produced by the respectively associated detector element, which may suitably comprise a photodiode. The magnitude of the stored charge signal represents the intensity of the x-ray falling on the detector element which caused the production of the charge.

The storage capacitors are connected together in a chain along the line defined by the row 3, 2, 1 of the detector elements, by circuitry described in more detail below.

Clocking circuitry 56, in response to signals from the encoder 36, provides clocking signals to each of the detector elements 3, 2, 1 such that in response thereto the charge signals are caused to iteratively shift to the left along the capacitors as shown by the arrow 58 in Figure 4A. Additionally, each time a charge is shifted one capacitor to the left, it becomes summed, or integrated, with any charge existing previously on the storage capacitor to which the charge is shifted. Thus, the clocking circuitry, in combination with the connecting circuitry and storage capacitors, iteratively shifts the charge packets on the storage capacitors and in the process accumulates the shifted charge with any charge which may have previously been held by the capacitor to which the charge is shifted.

The design and construction of the elements 3, 2, 1 and of the associated clocking, capacitor and connecting circuitry, will be described in detail below, but an exhaustive description here of those elements is believed to inordinately obscure this simplified example of detector operation.

Figures 4A-4D describe operation of the source-detector at four equally spaced points in time, i.e., $T_1$, $T_2$, $T_3$ and $T_4$. In the course of this operation, the source 10 and detector 14 are synchronously moved to the right as shown in Figure 4A at a predetermined average velocity. This motion may be continuous, or may be in stepwise fashion.

The charge shifting actuated by the clocking circuitry has an average velocity equal and opposite to that of the detector motion.

In this example, we shall consider imaging at only three points in the patient's body, i.e., along lines designated A, B, C in Figure 4A.

At time $t = T_1$, the detector is located such that x-ray energy penetrates the patient along the line A and falls incident upon the detector element 1. This occurrence results in the production of a charge $Q_{1A}$ on the capacitor $C_1$.

The charge designated as $Q_{1A}$ is that charge developed by individual detector element 1 as a result of x-rays passing through line A of the patient P. Charges developed on other detector elements by virtue of x-rays passing through other lines through the patient's body will be referred to by analogous notation.

Subsequent to the acquisition of charge $Q_{1A}$ on capacitor $C_1$, the detector moves, as shown in Figure 4B, to a location, at time $t = T_2$, at which x-rays passing through patient line B are incident upon detector element 1, and x-rays through patient line A are incident on detector element 2.

During the time of motion between the positions illustrated in Figures 4A and 4B, respectively, the clocking circuitry 56 has operated to shift the charge $Q_{1A}$ from capacitor $C_1$ to capacitor $C_2$. Now, upon arriving at the position shown in Figure 4B, capacitor $C_1$ acquires a new charge $Q_{1B}$, and capacitor $C_2$ also acquires a new charge referred to as $Q_{2A}$. However, since charge packet $Q_{1A}$ was already present on capacitor $C_2$ after the shift by the clocking circuitry, the total charge packet on capacitor $C_2$ is now $Q_{1A} + Q_{2A}$.

The process continues. The detector moves farther to the right such that at time t = $T_3$, the location is as shown in Figure 4C. Additionally, during the move between $T_2$ and $T_3$, the clocking circuitry 56 has again shifted the charges on all the capacitors by one element to the left.

It is now seen that x-rays from the source 10 are incident on each of detector elements 3, 2, 1 along lines A, B and C, respectively.

Since the previously held charge $Q_{1B}$ has been shifted from capacitor $C_1$ to capacitor $C_2$, capacitor $C_1$, just before time t = $T_3$, contains no charge. At t = $T_3$, however, capacitor $C_1$ acquires a charge $Q_{1C}$. Capacitor $C_2$ acquires a charge $Q_{2B}$ which is summed with charge $Q_{1B}$. Capacitor $C_3$, to which charge packets $Q_{1A}$ + $Q_{2A}$ have already been shifted, now acquires an additional new charge $Q_{3A}$.

Between time t = $T_3$ and time t = $T_4$, the clocking circuitry 56 again causes the iterative and accumulative shifting of charge by one capacitor to the left. This time, however, the charge reaching capacitor $C_3$, the last in the row, is transmitted to the data processing circuitry 44. The quantity of charge shifted, $Q_{1A}$ + $Q_{2A}$ + $Q_{3A}$ represents three accumulated charge packets, each of which is the result of a response of a different one of the elements 3, 2, 1 to x-ray energy passing through the same line A of the patient's body. Since exposure to each of the three elements 3, 2, 1 caused the production of the charge shifted to the data processor 44, that charge is approximately three times greater in magnitude than it would have been had only one exposure to a single element been used to provide data as to the radiation penetrating the patient along the line A, assuming equal detector scanning velocity. Thus, the signal to noise ratio of the charge signal is improved by a factor of about 3.

It will be noted that at time t = $T_4$, the charge now present on capacitor $C_3$ is $Q_{1B} + Q_{2B} + Q_{3B}$, and thus represents a value of charge produced in three exposures of three different elements in response to radiation passing through the same line B of the patient's body. Subsequently, the charge present on capacitor $C_3$ at time t = $T_4$ will also be transmitted to the data processor 44 for integration into the image representing data.

After the completion of a single scan, a signal from the encoder 36 causes the discharge of the storage capacitors C1, C2, C3 to prepare them for the next subsequent scan.

It can be seen from this example that, if the number of elements in the horizontal row were multiplied many times, the effect on the signal to noise ratio of the charge signals actually transferred to the data processing equipment would be improved still further.

Note that the processing circuitry 44 need not accept data simultaneously from all the elements. Rather, data is transferred only from capacitor C3, and only as rapidly as the clocking frequency of the shifting circuitry. This relatively slow data transfer rate enables the use of relatively simple and economical processing circuitry.

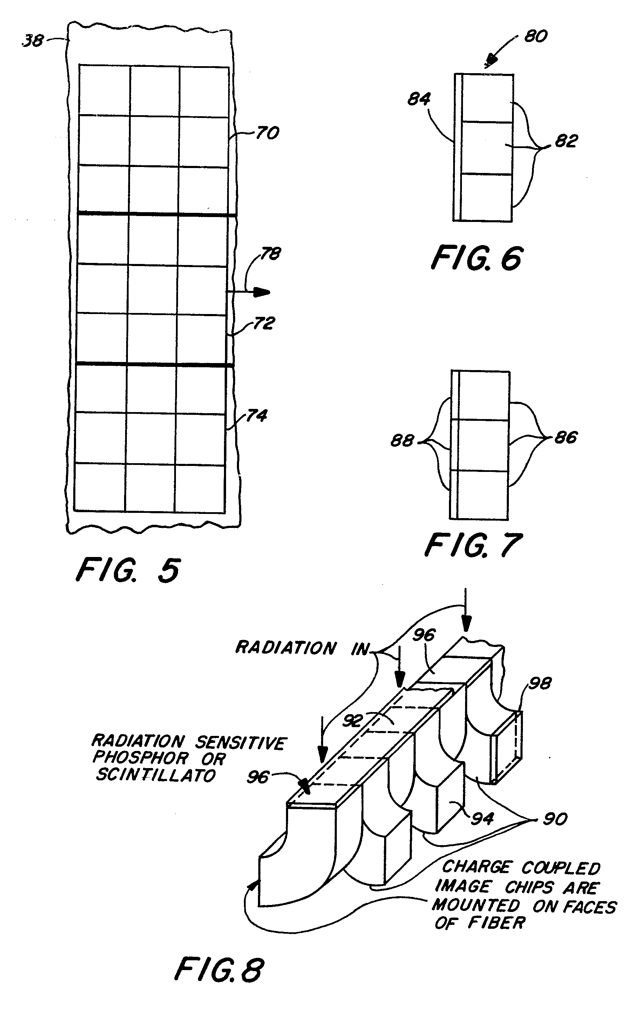

In the preferred embodiment, the detector assembly comprises a plurality of relatively large imaging chips, each of which includes a rectangular matrix of individual detector elements. Such an assembly of image chips, designated 70, 72, 74, is illustrated in simplified form in Figure 5.

The image chips are disposed in a vertical column, with respect to the system of Figure 1 and, as such, are moved during scanning in a direction illustrated by the arrow 78 in Figure 5.

Each of the horizontal rows of individual detector elements in each image chip is coupled to circuitry for causing their cooperative operation in a sequence analogous to that described in connection with Figures 4A-4D.

In Figure 5, each image chip is illustrated, for sake of simplicity, as comprising a 3 x 3 array of individual detector elements. In practice, and as described in more detail below, each image chip has an overall square configuration having a dimension of 8 millimeters on a side, and comprises either a 64 x 64 or 64 x 256 array of individual detector elements.

As mentioned above, in the preferred embodiment each individual detector element comprises a photodiode. The photodiode is responsive to visible light falling upon a receiving surface to produce an electrical charge signal representing a characteristic of the visible light which caused the production of the signal.

Figures 6-8 illustrate various structures for interfacing the visible light sensitive photodiodes with incident x-ray energy emergent from the patient's body.

Figure 6 shows a side view of 3 x 3 image chip 80 including photodiodes 82. Overlying the photodiodes 82 is a phosphor layer 84 made of one of the known materials for converting incident x-ray to visible light. When x-rays impinge from the left as shown in Figure 6, the phosphor layer 84 luminesces and the resulting visible light pattern is detected by the individual detector element photodiodes 82.

Sometimes it is desirable to provide protection from excessive exposure of the silicon photodiodes to direct x-radiation. A structure suitable for such purpose is illustrated in Figure 7.

Figure 7 is another side view of an image chip illustrating three photodiodes 86. In this embodiment, the phosphor layer of Figure 6 is replaced by a mosaic of individual scintillation crystalline material illustrated at reference character 88. The crystalline material can suitably comprise thallium activated sodium iodide or some other suitable crystal known to those of ordinary skill as having the property of converting x-radiation to visible light. The crystals 88 can be somewhat thicker than the phosphor layer 84 shown in Figure 6. The thicker crystals are more absorptive of radiation than is the phosphor layer, and thereby provide some shielding effect for the photodiodes, while still facilitating their operation. -

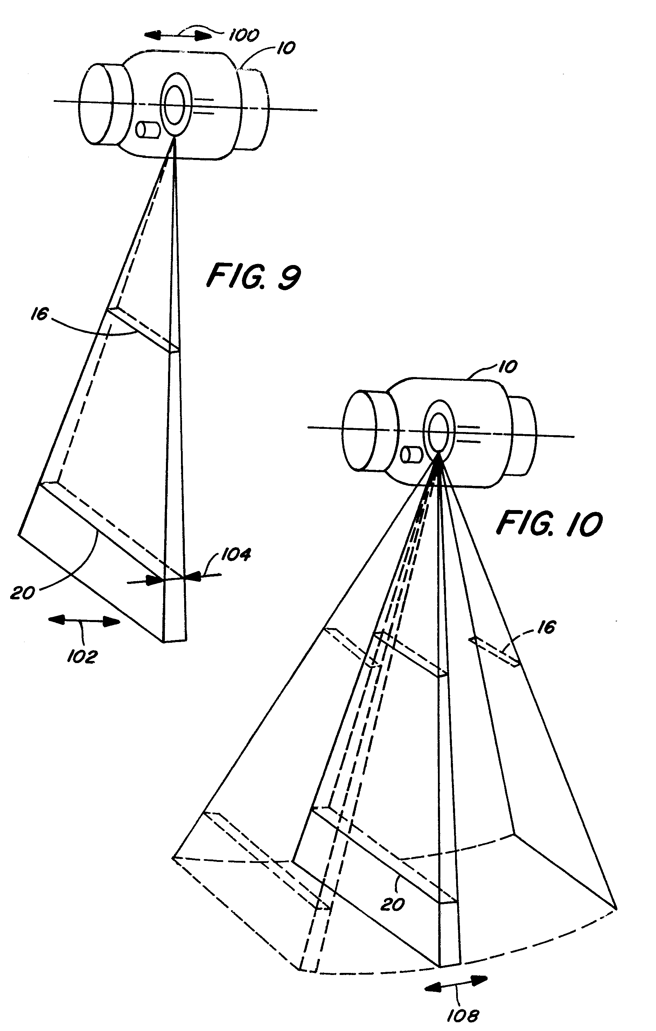

Another arrangement, providing still greater radiation protection for the image chips, is shown in Figure 8. The principal feature shown in Figure 8 is a series of coherent fiber optic coupling elements illustrated for example at reference character 90. Each optical coupling element has an input face such as 92 and an output face such as shown at reference character 94. The input face and output face 92, 94 are configured to be geometrically congruent with the square face of each of the imaging chips. Suitable fiber optic materials include quartz, glass and plastic.

A phosphor layer, such as 96, overlies each of the input faces of the coupling elements 90. The output face of each coupling element is optically coupled to the receiving face of an image chip such as shown at reference character 98.

By the use of coherent fiber optic coupling, the image chips can be disposed at a location which is not in the path of x-radiation from the source 10, or at a location at which its receiving face is oriented in a direction such that the incident radiation from the source does not fall upon that surface.

The embodiment of Figure 6 is preferable where cost is paramount, and is suitable for uses such as in baggage inspectors.

Figures 9 and 10 illustrate certain hardware implementations for achieving the moving spread beam. In Figure 9, for example, both the x-ray source 10 and the detector assembly are translated along parallel linear paths indicated by arrows 100, 102 in Figure 9. Tests suggest that a preferable thickness for the spread beam is 8 millimeters at the input surface of the detector elements, but a beam from 1 to 50 millimeters in thickness can be used. This dimension is indicated by arrows 104 in Figure 9.

The use of a single slit, relatively thick fan beam is believed preferable to the use of multiple slits because a single slit exhibits less penumbra effect than does a multiple slit embodiment.

Another hardware implementation is illustrated in Figure 10. In this embodiment, the detector moves generally along an arcuate path indicated by arrows 108 in Figure 10. The source 10 can be pivoted about its x-ray focal spot to cause the x-ray beam to track the detector. Alternately, where the cone of x-rays generated by the x-ray tube is sufficiently divergent, the x-ray tube may be retained in a stationary disposition, the actual x-ray energy striking the detector being collimated by the collimating slit defining structures 16, 20, illustrated in Figures 9 and 10.

In accordance with a third possible embodiment, the concept of this invention may be employed by maintaining the x-ray source and the detector both stationary, while the subject for examination moves along a linear path between the source and detector. Such an embodiment could be suitable, for example, in a baggage inspection system.

A requirement peculiar to the system embodying this invention is that data is acquired in one x-ray spread beam scanning direction only. This direction is from left to right, as shown in Figure 1. The mechanical scanning apparatus should be capable of rapid retrace between production of sequential images. It is mechanically practicable to produce retrace at velocities much higher than scanning velocities.

Tests have shown that suitable scanning velocities at the detector face are in the neighborhood of up to about 26 cms per second.

In embodiments employing a moving spread beam of x-rays, it has been found preferable that the detector itself should be coupled to the master scanning drive mechanism. The source, and the slit defining collimators should be the follower elements, since their instantaneous position is less critical than that of the detector. An encoder, such as shown in Figure 1, coupled to the detector, provides timing pulses to control the position of the source and/or collimating slit defining structures. The pulses output from the encoder are also coupled to the clocking circuitry 56 to control the timing of charge stepping of the charge shifting and accumulating circuitry. Such an arrangement will assure good synchronization between detector motion in one direction and charge motion in the opposite direction at the same average velocity. Very accurate synchronization of charge and detector motion is possible since the moving charges on the detector do not possess inertia and the charge shifting can be implemented with a favorable frequency response.

Retrace and scanning rates, and hence frame repetition rate, is substantially limited by only the mechanical considerations, not by optics or electronics. Tests have shown that frame repetition rates of about 30 per minute are feasible, using a frame size of 50 cm. x 50 cm.

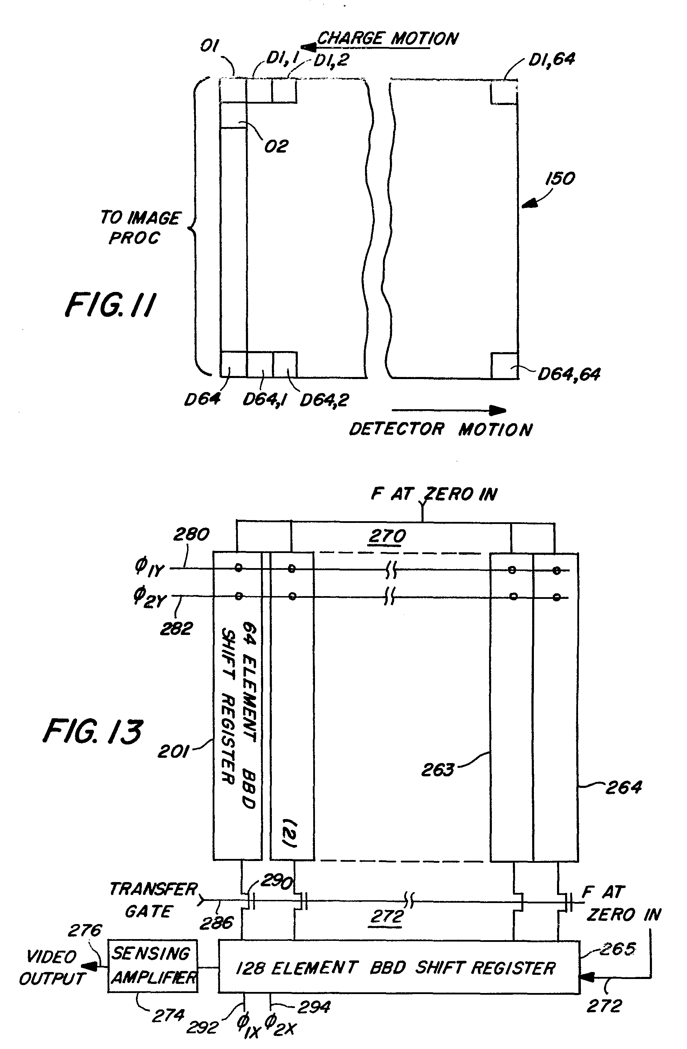

Figure 11 shows in more detail an embodiment of one of the imaging chips, indicated at reference character 150. In the Figure 11 embodiment, the image chip comprises a 64 x 64 rectilinear array of individual detector elements, each comprising a photodiode. The detector elements are arranged in 64 rows and columns.

For example, one such row comprises individual detector elements D1,1-D1,64. Sixty-four rows of detector elements are present on the imaging chip, extending downwardly to the row comprising individual detector elements D64,1-D64,64.

A shift register including cells O1-O64 is coupled in parallel to the column of detector elements D1,1-D64,1.

In use, the image chip, and the detector as a whole, move to the right as indicated by the arrow in Figure 11. During scanning motion, the shifting and integration circuitry cause accumulating charge motion in a direction to the left as shown in Figure 11, synchronously with the detector motion in the opposite direction. When the accumulated charge packets, each representing projections of the same area of the patient's body, have been so integrated and transferred and have reached the respective individual detector elements of column D1,1-D64,1, the charge packets are transferred in parallel to the shift register, i.e., into each of the cells O1-O64. The data thus accumulated can be clocked out, digitized, and processed to yield image representations in a manner known in the art. Systems for producing imaging data from sequentially occurring line-representing sets of data derived from a photodiode array are described in U.S. Patent No. 4,203,037, issued May 13, 1980 to Gur et al, particularly with respect to columns 6 and 7 of that patent, and the patents and publications incorporated therein. Patent No. 4,203,037, and all other patents and publications incorporated therein are specifically incorporated by reference here.

The 64 x 64 array of individual detector elements of the image chip 150 of Figure 11 collectively describe a square of 8 millimeters length on a side. As noted above, the detector 14 comprises a vertical column of such imaging chips, each being 8 millimeters wide, and the spread beam incident on the detector is vertically oriented and 8 millimeters in thickness, just sufficient to illuminate the entirety of the vertical column of imaging chips.

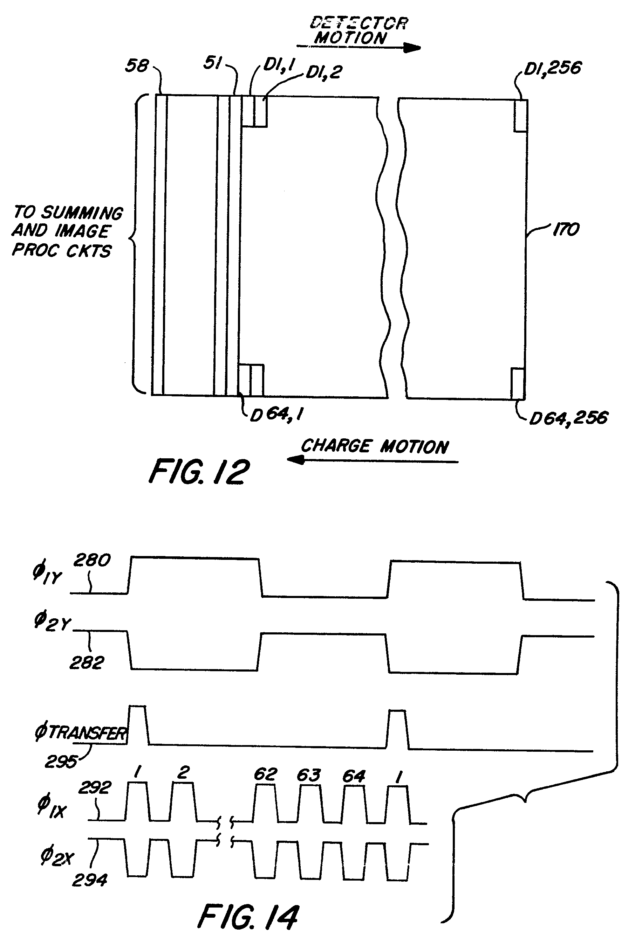

Figure 12 illustrates another embodiment of an image chip indicated by the reference character 170. The image chip 170 comprises a 64 x 256 array of rectangular individual detector elements. The array comprises 64 horizontal rows of 256 elements each. Each individual element has a horizontal width which is approximately 1/4 its vertical height. Together, the 64 x 256 detector array describes a square approximately 8 millimeters on a side.

As in the embodiment of Figure 11, the detector, during scanning, is moved to the right as shown in Figure 12, and the charge packets are shifted and integrated to the left, at a rate synchronous with that of detector motion.

The embodiment of Figure 12 is considered particularly suitable for the use of time delay and integrate circuitry consisting of CCD elements. The 64 x 64 array of Figure 11 is considered more suitable for the use of bucket brigade circuitry which can handle wider charge "bins", and hence has a greater capability than CCD for shifting charge packets efficiently. Efficiencies of at least about 0.99995 are desirable.

In operation, the charges are shifted to the left, as shown in Figure 11 in synchronism with detector scanning motion. Data respectively corresponding to projections of the same image line is integrated and transferred along each element until the charge reaches the extreme left-hand detector element in the leftmost column of detector elements, i.e. D1,1-D64,1. At that point, the charge packets are transferred and clocked successively through a series of parallel shift registers S1-S8. The clocking through the registers S1 through S8 is at the same rate as the clocking along the various detector elements. No integration, however, takes place in the shift registers.

Preferably where, as in the case of the Figure 12 embodiment, the individual detector elements are only 1/4 as wide as they are tall, circuitry of known type is provided which, in conjunction with the shift registers S1-S8, periodically sums output data in the shift registers S1-S8. After each succession of four shifts, the data in S1-S4, corresponding to each separate element row, is separately summed in appropriate output register circuitry, and then clocked out to digitization circuitry and image processing circuitry such as described in connection with Figure 11.

Figure 13 is a block diagram showing circuitry of the matrix array of an image chip in greater detail.

The image chip shown comprises a 64 x 64 element bucket brigade matrix array for detecting x-rays after they have been converted to green light, similar to that of Figure 11. The matrix array is operated in a time delay integration mode. The center-to-center spacing of the individual detector elements in both the column and row directions is 5 mils. The array is integrated using a double poly-silicon gate NMOS process. The design details and dynamic range consideration of the image chip array are discussed in detail below.

The image chip includes 64 horizontal bucket brigade (BBD) shift registers 201-264. Also included is a 128 element BBD shift register 265.

Each of the registers 201-265 is provided with a fat-zero input port (designated generally by reference characters 270, 272) for enhancing charge transfer efficiency in the registers.

Registers 201-264 perform the time delay and integrate (TDI) function while register 265 transfers the signal charge package to an output sensing amplifier 274. The output sensing amplifier converts the charge to a signal voltage appearing at a lead 276 which is a function of the charge input to the amplifier. The signal at the lead 276 provides a video output.

The center-to-center spacing of the registers 201-264 is 5 mils. Each of the registers 201-264 comprises 64 BBD elements (32 stages) and is driven by a two-phase complementary clock having inputs designated by the reference characters 280, 282.

Each BBD element is constructed with a diffused photodiode and a poly-silicon-to-poly-silicon storage capacitor. The sensing area of each photodiode is approximately 50% of the total area of the individual detector element receiving surface. The capacitance of each individual detector element is about 1.2 picofarads (pF).

When light falls upon each photodiode, photoelectrons are generated. This photocharge is stored on the storage capacitor. The two-phase clocks at the leads 280, 282 shift the charge packages along each of the shift registers 201-264 toward the register 265. In the TDI operation, the image velocity across the image chip is synchronized to the shift velocity of the charge package (integration site). The clocking rates of the signals at the leads 280, 282 is approximately 0.5 kilohertz (kHz). The total integration time of each pixel is approximately 64 milliseconds (msec).

This relatively slow integration time allows a tolerance for x-ray tube rotor and voltage ripple, and substantially reduces their artifacts.

When the charge packages reach the ends of the registers 201, 264 adjacent the register 265, a transfer pulse is applied to a transfer gate 295. This activates the transfer switches, shown for example at reference character 290. The signal charges are thus transferred in parallel to the register 265 from each of the registers 201-264.

The register 265 is driven by a two-phase clock having inputs 292, 294. This clock signal runs at a frequency 64 times faster than that of the clock signals at the leads 280, 282.

The output sensing amplifier of the register 265 is a gated charge integrator which converts the signal charges into output voltages.

The timing diagram of the clocks is illustrated in Figure 14.

The clock waveforms 280, 282 and 292, 294 are complementary clocks, cross over at 50%. The frequency $f_x$ = 64 X $f_y$ = 32 kHz. The rising edge of waveform 292 leads that of waveform 295 by about 30 nanoseconds (ns.) and waveform 295 leads waveform 280 by about 20 ns. The falling edge of waveform 295 leads that of waveform 292 by at least 30 ns.

The charge handling capacity of the BBD elements depends upon the size of the storage capacitor and of the clock voltage. Assuming a maximum voltage swing of 5 volts at each BBD storage node, the saturation charge of each BBD element will be about 6 picocoulombs (pC), i.e., 1.2 pF x 5 v. This saturation charge is equivalent to a signal charge of 3.75 x $10^7$ electrons.

There are two noise components in the signal output from the sensing amplifier. One is the fixed-pattern noise caused by nonuniformity of the input fat-zero among the registers 201-264. This noise component can be eliminated by external signal processing. Therefore it is not the limiting factor in the performance of the device.

The other noise component is the thermodynamic noise. This noise consists of input kTc.noise, BBD transfer noise, dark current shot noise, photo signal shot noise and output amplifier noise. However, the dominant thermodynamic noise is BBD transfer noise.

At room temperature, the noise electron charge, due to this component, is approximately 6000 electrons. This noise figure results in a dynamic range of about 6250 ($3.75 \times 10^7/10^3$) for the device.

The calculated objective specifications of the matrix array is listed in Table I.

### TABLE I

Calculated Objective Specification of the Matrix Array.

| | |
|---|---|
| No. of Elements | 64 by 64 |
| Center-to-Center Spacing | |
| X Direction | 5 mils |
| Y Direction | 5 mils |
| Element Storage Capacitance | 1.2pF |
| Element Sensing Area | 50% of total element area |
| Saturation charge | 6 pC |
| Thermodynamic Noise | |
| Electron | $6 \times 10^3$ electrons |
| Dynamic Range due to Thermodynamic noise | 6000 |
| Saturation Output | Approx. 4V |

An example of time delay and integrate circuitry for use in imaging is described in the following article, which is expressly incorporated by reference: Milton, A. F. "Charge Transfer Devices for Infrared Imaging", pp. 197-228, which is a portion of the book "Optical and Infrared Detectors" edited by Keyes, R.J. (1977).

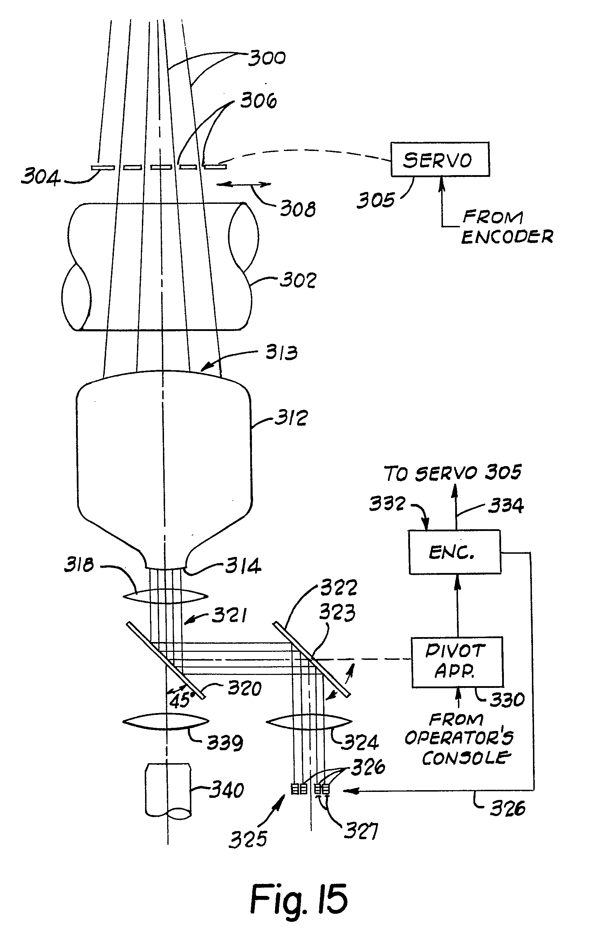

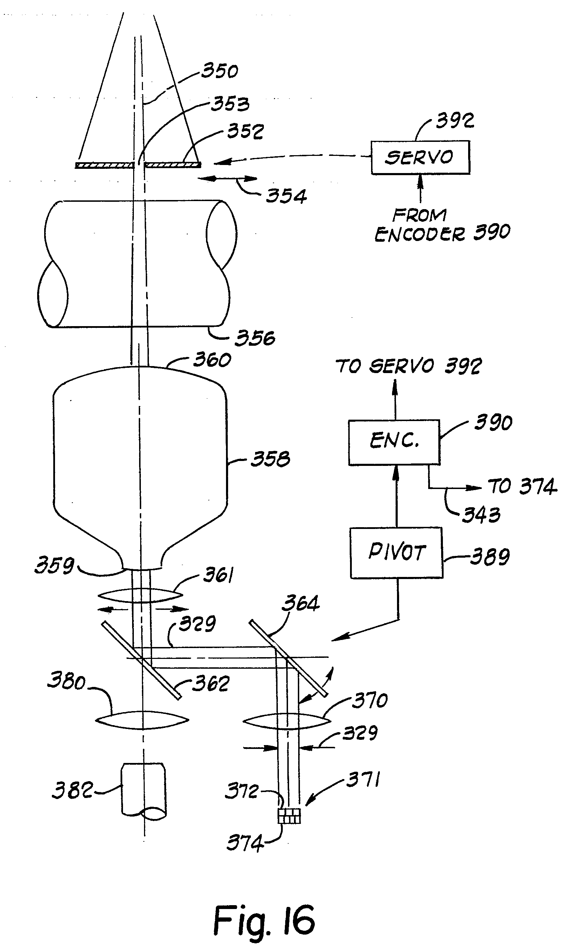

Figures 15 and 16 illustrate other embodiments of a digital radiographic system utilizing time delay and integrate (TDI) techniques in scan projection radiography. These alternates possess an enhanced frame generation rate capability, and couple the use of area detectors with discrete element charge coupled imagers operating in the TDI mode.

Referring to Figure 15, x-rays from a source (not shown) and defined by ray lines 300 are directed toward a subject 302. The x-rays are directed generally toward an approximately cylindrical image intensifier tube 312, and more particularly toward an input face 313 of the image tube. The x-ray pattern incident upon the image tube causes the image tube to respond by generating a corresponding smaller, relatively bright visible light pattern at an output face 314.

A slit collimator 304 is interposed between the source and the subject. The slit collimator is made of a material which is substantially impervious to x-rays, and in which is defined a plurality of slits such as indicated by reference character 306.

The slit collimator is mounted for movement transverse to the incident x-rays in the directions indicated by the arrow set 308 in Figure 15. A servo mechanism 305 is coupled to the slit collimator 304 to effect this movement in a fashion described in more detail below.

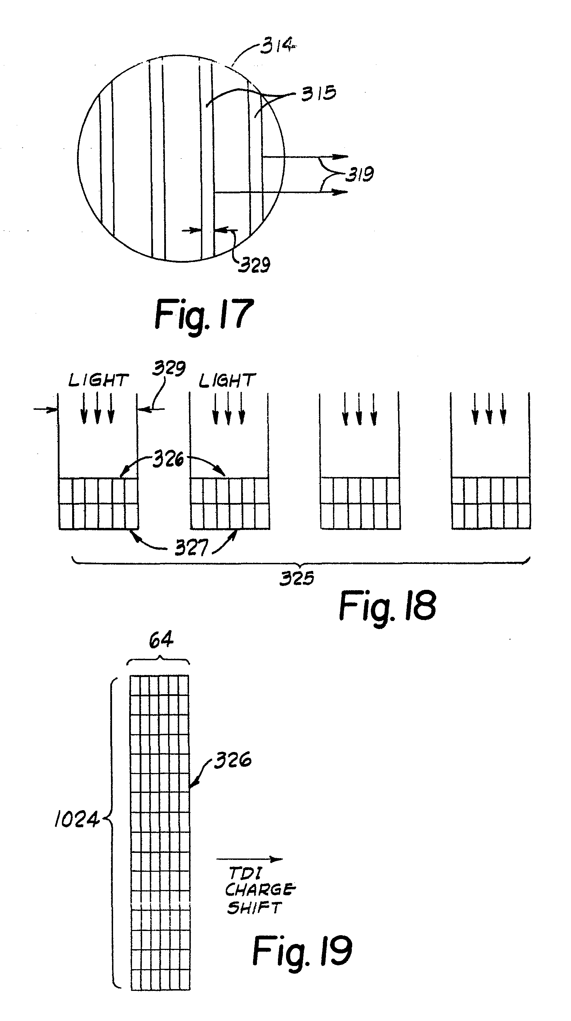

The x-rays, collimated into slits by the collimator 304, pass through the subject and fall incident upon the image tube input face as slit x-ray patterns lying substantially within respective planes which are perpendicular to the paper relative to Figure 15. The incidence of the x-ray slit patterns causes the image tube 312 to produce a plurality of visible light slit patterns 315 (see Figure 17) at the output face 314. One such light slit pattern is produced corresponding to each x-ray slit pattern falling upon the input face 314. The light slit patterns appearing at the output face 314 lie along respective axes which are perpendicular to the paper with respect to Figure 15. The light patterns have a width designated by reference character 329 (Figures 17 and 16).

When the slit collimator 304 is moved from left to right in Figure 15, the slit light patterns 315 move from left to right across the output face 314, as shown by the arrows 319 in Figure 17.

Each of the slit light patterns appearing at the output face 314 is projected through a collimating lens 318 and onto the surface of a partially transmissive mirror 320. The mirror 320 is a plane mirror extending perpendicular to the paper as viewed in Figure 15. The mirror 320 is oriented at approximately 45° with respect to the central axis of the image intensifier tube 312.

As illustrated in Figure 15, more specifically at lines 321, each of the slit light patterns strikes the mirror 320 at a slightly different location along its surface. The slit light patterns are projected horizontally as shown in Figure 15 onto a second planar mirror 322. The mirror 322 is mounted pivotally with respect to an axis 323 extending perpendicular with respect to the paper as shown in Figure 15.

The slit light patterns are reflected from the mirror 322 through a second collimating lens 324 and optionally through other optical elements, not shown

but suitably provided by one of skill in the art, onto a detector assembly 325 comprising a set of detector arrays 326. The detector arrays 326 are stationary with respect to the image intensifier tube 312, and with respect to the mirror 320.

The detector arrays 326 are equal in number to the number of slit light patterns projected toward the detector 325. See Figure 18 for detail.

Each of the detector arrays 326 suitably comprises a separate rectangular array of small individual detector elements. The rectangular configuration of each array is elongated along an axis perpendicular to the paper in Figure 15. More specifically, tests have shown that a suitable embodiment for each of the detector arrays 326 comprises either a 1024 X 64 or 2048 X 64 array of individual detector elements (see, e.g., Figure 19).

Each of the detector arrays 326 is constructed analogously to the detector array embodiments described in simplified form in connection with Figure 5 and in more detail in connection with Figures 13 and 14.

Coupled to each detector array 326 is a system 327 of time delay and integrate circuitry. This circuitry and its operation is analogous to that discussed above in connection with Figures 4A-4D, 5, 13 and 14.

A pivot apparatus 330 is coupled to the pivotable mirror 322. In response to a command signal from the operator's console, the pivot apparatus 330 operates to pivot the mirror 322 in a manner described in more detail below. The pivot apparatus 330 is coupled to an encoder 332. The encoder 332 senses the rotational position of the mirror 322, as determined by the pivot apparatus 330, and produces a signal at a lead 334 representing substantially the instantaneous rotational orientation of the mirror 322. The signal at the lead 334 is transmitted to the servo mechanism 305 in order to synchronize the translational movement of the slit collimator 304 along the path indicated by the arrows 306. The nature of this synchronization is described more fully below.

Another output from the encoder 332 is transmitted over a lead 336 to the time delay and integrate circuitry 327 associated with each detector array 326. The signal at the lead 336 from the encoder 332 synchronizes the clocking circuitry of the time delay and integrate circuitry in order to synchronize the charge shifting function with the angular velocity of the pivotable mirror 322 and the translational velocity of the slit collimator 304.

The encoder 332 can suitably comprise any of a plurality of known types of such devices, for example, the encoder apparatus and circuitry described in the above incorporated United States Letters Patent to Manring.

In operation, the pivoting motion of the mirror 322 and the translational motion of the slit collimator 304 are synchronously coordinated in order to maintain the projection of each of the slit light patterns propogated from the output face 314 stationary with respect to the detector assembly 325, notwithstanding the motion of the slit light patterns across the output face 314. As can be seen from the illustration of Figure 15, in order to accomplish this function, a motion of the slit collimator from left to right must be accompanied by a corresponding pivoting of the mirror 322 in a clockwise direction. The ratio of the translational velocity of the slit collimator 304 to the angular velocity of pivoting of the mirror 322 in order to maintain the stationary projection of each of the slit light patterns on the detector 325 is determined by the particular geometry and optics of the system, and can readily be determined by one of ordinary skill given the above system design parameters.

Where the multiple slit system of Figure 15 is employed, and the slits of the collimator 304 are distributed such that the slit x-ray patterns are equally spaced across the entire input face 313 of the image tube 312. Inspection of Figure 15 will show that, in order to scan the entirety of the input face 313, it is necessary to translate the slit collimator 304 in an amount equal to only the distance between adjacent slits.

The translation of the slit collimator may be over the whole width of the input face, depending, as illustrated for example below, on the system configuration.

As mentioned above, signals from the encoder 332 are utilized to clock the time delay and integrate circuitry 327 associated with each of the detector arrays 326. The direction and average clocking velocity of the charge packets produced by the elements 326 is a function of both the velocity and direction of translation of the slit collimator 304 during scanning.

The charge shifting is in a direction opposite in sense to the direction of movement of the slit light patterns across the output face 314 of the image tube. More specifically, if scanning is conducted with the system of Figure 15 and with the slit collimator 304 moving in a direction from left to right, the direction of clocking of the charge shifting motion is to be in a right to left direction as illustrated with respect to the detector 325.

In the preferred embodiment the width of each of the slits of the collimator 304 is chosen such that the projections 329 (see Figure 18) of the slit light patterns are each of a width equal to that of one of the detector arrays 326.

The average velocity of the clocking of charge shifting motion is, under these conditions, chosen to be a velocity such that charge is shifted across the entirety of the width of one of the detector elements 326 in the same amount of time which is required for a slit light pattern appearing at the image tube output face 314 to traverse a distance equal to its own width.

It is important to note that, when the system of Figure 15 is operated in the described mode, with the slit collimator 304 moving to the right, and the mirror 322 pivoting in a clockwise direction, the image "seen" by each stationary detector array 326 is a continuous view of one of the slit light patterns at the output face 314 as it moves across the face. This condition is equivalent to a situation in which slit light pattern is moved across the face 314, with the detector array being also moved at the same velocity to remain in continuous optical alignment with the slit pattern. Thus, it is seen that time delay and integrate techniques analogous to those described above (for example in simplified form in connection with Figures 4A-4D) can be employed.

Referring still to Figure 15, the partially transmissive mirror 320 comprises a form of beamsplitter. The portion of light energy from the output face 314 which is transmitted through the mirror 320, is propgoated through a lens 339 and onto the input 340 of a video system. the video system can comprise any of a number of such systems known in the art as suitable for use in fluorography applications where an image intensifier tube is employed. Accordingly, the remainder of the video system is not illustrated.

By the removal or translation of the slit collimator 304 entirely out of the path of the x-rays propagating toward the input face 313, the system of Figure 15 can be operated as a conventional bright fluoroscopic system. Conversely, a conventional fluoroscopic system can be converted to add digital radiographic capabilities such as described above by adding the movable slit collimator 304, pivotable mirror 322, detector assembly 325, and their associated mechanical and electronic synchronization equipment as shown and described in connection with Figure 15.

Figure 16 illustrates another embodiment, similar, but not identical, to that of Figure 15.

It is not necessary to employ two mirrors. In an alternate embodiment, the detector and lens arrangement could be reoriented to directly view the light pattern from the partially transmissive mirror, and that mirror could be arranged to pivot. In such a case, the second mirror could be omitted.

In Figure 16, x-rays, denoted by reference character 350, are directed toward a slit collimator 352. In the embodiment of Figure 16, the slit collimator defines a single slit 353 having its longitudinal dimension extending perpendicular to the paper as shown in Figure 16. X-rays passing through the slit 353 are directed through a subject 356 and onto an input face 360 of an image intensifier tube 358. The single slit x-ray pattern falling on the input face 360 causes the intensifier tube to generate at an output face 359 a single slit light image representing the x-rays of the slit pattern falling upon the input face.

An image of the slit light pattern is projected through a collimating lens arrangement 361 and onto a beam splitter comprising a partially transmissive mirror 362. The mirror 362 has a planar configuration similar to the mirror 320 in Figure 15.

The image of the single slit light pattern is projected onto a second planar mirror 364 which is pivotally mounted about an axis 366. The image reflected from the mirror 364 is projected through a lensing arrangement 370 and onto the input of a detector 371.

The detector 371 comprises a single rectangular pattern of individual solid state discrete detector elements, arranged in an elongated pattern similar in configuration to that shown in Figure 19. As in the case of the previously described embodiment, the rectangular element array can suitably comprise a 64 X 1024 or a 64 X 2048 array of elements. In a preferred embodiment, the width of the detector array 372 is approximately 8 millimeters.

Time delay and integrate circuitry 374 is coupled to the respective transverse rows of individual detector elements of the array 372. The detector elements of the array 372 and the time delay and integrate circuitry 374 are analogous in structure and operation to that described in connection with Figure 15.

An important difference between the embodiments of Figures 15 and 16 is that the embodiment of Figure 16 uses a slit collimator having only a single slit, and produces at the output face 359 a single slit light pattern. Since only one slit light pattern is generated, only the one array of individual detector elements 372 is required for the detector 371.

The pivotally mounted mirror 364 is coupled to a pivot apparatus 389 which serves to controllably pivot the mirror about its axis 366. The pivot apparatus 389 is responsive to a command signal from the operator's console to so pivot the mirror. An output from the pivot apparatus 389 is coupled to an encoder 390 which produces a signal indicating substantially the instantaneous angular displacement of the mirror 364 about its axis 366. An output of the encoder 390 is directed to a servo apparatus 392. The servo apparatus 392 is coupled to the slit collimator 352 in order to move the slit collimator to the right and left, as commanded, and as illustrated by the directions indicated by the arrows 354.

The operation and construction of the servo 392, encoder 390 and pivot apparatus 389 is as described in connection with the operation of the servo 305, encoder 332, and pivot apparatus 330 in connection with Figure 15.

As in the embodiment of Figure 15, the encoder 390 produces another output on a lead 393 which is directed to the time delay and integrate circuitry 374 of the detector 371. The circuitry and functioning of the encoder with respect to the time delay and integrate circuitry 374 is analogous to that described in connection with Figure 15. That is, the signal from the encoder clocks the time delay and integrate circuitry to shift the charges generated by the individual detector elements in a direction opposite in sense to the direction of movement of the slit light pattern across the output face 359.

The second mirror of Figure 16 can be omitted in a manner analogous to that described in connection with the Figure 15 embodiment.

Some of the technical problems associated with the embodiment of Figure 15 are overcome or reduced by use of the embodiment of Figure 16. For example, high data rates are reduced by lumping the slits together into a single slit pattern feeding information to a single detector array. The effects of pin cushion distortion are reduced. While such distortion is still present, it may be corrected by proper lens design, as can be done by one of ordinary skill in the art.

Additionally, the effects of scatter are reduced by use of the single slit embodiment.

The embodiments of Figures 15 and 16 are feasable for operation without slit collimators interposed downstream of the image tube output face. However, scatter rejection can be further improved if an appropriate slit collimator were employed just ahead of the discrete solid state detector.

It is also feasible to employ a scatter grid on the image tube to further reduce scatter effect.

In the embodiments of Figures 15 and 16, the longitudinal dimensions of the individual arrays of discrete solid state detector arrays are designed to be sufficiently tall to accommodate the full length of a slit light pattern appearing at the output face of the intensifier tube.

Another advantage of the embodiments of Figures 15 and 16 is that optics associated only with the branch of the system directed toward the TDI detectors can correct for pin cushion distortion and can also control the amount of light incident on the solid state detector, independently of influencing light directed toward the video portion of the system.

The output of the solid state detector can be transmitted directly to a digital camera to provide a hard copy. Accordingly, the system of Figures 15 and 16 can be employed to do fluoroscopic examinations utilizing the video branch of the system, while still having capability of providing high quality digital scans utilizing the discrete solid state detector portion.

It is to be understood that the description of this invention set forth above is intended to be illustrative, rather than exhaustive, of the invention. It is to be recognized that persons of ordinary skill in the relevant technical field will be able to make certain additions, deletions and modifications to the embodiments described herein without departing from the

0166567

spirit or the scope of the invention, as defined in the appended claims.

Claims

1. An imaging system characterised in that it comprises: a source of penetrative radiation (300); an area detector (312 or 353) having input (313 or 360) and output (314 or 359) portions; means for scanning radiation (300) from the source through a subject (302) and across the input portion (313 or 360) of the area detector (312 or 353) in a pattern covering at one time only a fraction of the input portion of the area detector, causing the area detector to produce at its output portion a moving representation of the radiation being scanned across its input portion; a second detector (325); means (322,323, 324) for projecting the moving representation of scanned radiation continuously onto a fixed portion (326) of the second detector and imaging circuitry coupled to the second detector (325 or 371) for producing an image representing internal structure of the subject.

2. An imaging system according to Claim 1 in which the penetrative radiation is x-radiation.

3. An imaging system according to either of the preceding Claims, wherein said second detector (325) is fixed in position with respect to said area detector (312).

4. An imaging system according to any one of the preceding Claims, wherein said imaging circuitry comprises time delay and integrate circuitry (327 or 374).

5. An imaging system according to any one of the preceding claims including transmission circuitry for restoring spatial definition to the imaged portions presented to the second detector using second detector output data, in a temporal sequence corresponding to the temporal scanning sequence of the radiation.

6. An imaging system according to any one of the preceding claims in which the means for scanning radiation (300) from the source comprises a structure (304 or 352) defining a slit (306 or 353) interposed between the radiation source and the subject (302 or 356); and a mechanism for moving the slit defining structure (304 or 352) transverse to the radiation path (300 or 350) in order to scan a slit pattern of said radiation across the input portion (313 or 360) of the area detector to produce a moving slit pattern within one representation at said output portion (314 or 359).

7. An imaging system according to any one of the preceding claims in which the area detector (312 or 315) is an image intensifier tube, the moving representation being an optical representation.

8. An imaging system according to Claim 7 including a third detector (340 or 382) comprising a video camera, and means (320) for optically coupling the output portion (314 or 359) of the area detector (312 or 315) to both the second (325 or 371) and third detector (340 or 382).

9. An imaging system according to Claim 7 or Claim 8 including a pivotable mirror system (320, 322 or 362, 364) positioned to intercept light from the output portion of the image intensifier tube; apparatus (330 or 389) for pivoting said pivotable mirror system (320, 322 or 362, 364) and synchronization apparatus and circuitry (390, 392) coupled between said pivotable mirror system (320, 322 or 362, 364) and said movable slit defining structure for synchronizing the transverse movement of the slit defining structure with the pivoting motion of the pivotable mirror system to continuously direct and maintain the moving light from said output portion (314 or 359) incident continuously upon substantially said fixed portion of said second detector (325 or 371).

10. An imaging system according to any one of the preceding claims in which said second detector comprises a rectilinear matrix of individual detector elements having a plurality of rows of detector elements.

11. An imaging system according to Claim 10 when dependent on Claim 4 in which the time delay and integrate circuitry (327 or 374) is coupled to said respective rows of individual detector elements, the time delay and integrate circuitry including circuitry for shifting and integrating charge generated by said individual detector elements along each row of detector elements; the time delay and integrate circuitry (327 or 374) being clocked such that said charge shifting across said detector element rows (326 or 372) is dependent upon said movement of said representation across said area detector (312 or 353) output portion (314 or 359).

12. The system of Claim 1 wherein said time delay and integrate circuitry (327 or 374) comprises clocking circuitry for shifting said charge in a sense which is opposite in direction to the direction of movement of said representation.

13. The system of Claim 12 wherein said clocking circuitry shifts said charge at a rate which is a function of the average velocity of movement of said representation.

14. An imaging method comprising: scanning penetrative radiation relative to a subject to collectively define a single area pattern of radiation emergent from the subject; scan imaging in a temporal sequence a succession of spatially distinct portions of the pattern; the method being characterised in that it includes the steps of presenting the temporal sequence of the spatially distinct imaged portions of the pattern to a single spatially non-varying portion of a detector input to produce detector output signals; restoring spatial definition to the portions presented to the spatially non-varying detector portion by removing detector output signals produced in response to said presenting step in a second temporal sequence corresponding to the temporal sequence in which the spatailly distinct succession of x-ray pattern portions were imaged in said imaging step; and utilizing the second temporal sequence of removed detector data to

reconstruct a consolidated image corresponding to the succession of spatially distinct x-ray pattern portions imaged in said imaging step, whereby an image corresponding to all the imaged pattern portions is produced by a detector having a field size sufficient to accommodate only one of the imaged pattern portions at a time.

15. The method of Claim 14, wherein the detector utilized comprises rows of individual detector elements defining a rectangular array, and time delay and integrate circuitry coupled to respective rows of the individual detector elements, said spatial definition restoring step comprising: clocking the time delay and integrate circuitry to shift and accumulate charge signals along said rows in a directional sense opposite that of the temporal sequence of imaging of successive spatially distinct x-ray pattern portions; and removing accumulated and shifted data from the detector as it reaches end elements of said rows.

16. The method of Claim 15, wherein said clocking step comprises: shifting and accumulating detector element signals at a scalar average velocity which corresponds to the average scanning velocity of said x-rays.

17. An imaging method utilizing a source for directing penetrative radiation (300 or 350) through a subject (302 or 356), a first area detector aligned to receive radiation emergent from the subject, the first detector being responsive to such emergent radiation over a field to produce a representation of the pattern of such emergent radiation over the field, and a second detector (325 or 371), said method being characterised in that it comprises the steps of: inhibiting exposure of said area detector to radiation over all but a predetermined time variable portion of the field of said first detector, such that the representation produced by the first detector is only of radiation corresponding to the time varying portion of the field of the area detector; continuously transmitting said positionally time varying representation to said second detector during said time variance along a path which is, at the point of incidence on said second detector, stationary with respect to said second detector; sampling the output of said second detector over time; and employing said sampled output information to produce a tangible representation of internal subject structure.

18. An imaging method according to Claim 17 in which the pattern representation produced by the area detector (312 or 353) is in the form of visible light; and said transmitting step comprises: reflecting the representation off a pivotable mirror system (320, 322 or 361, 364) and pivoting the mirror system in synchrony with said positional time variance to maintain an image of the visible light representations directed onto a uniform portion of the second detector (325 or 371).

19. An imaging method according to any one of Claims 14 to 17 including the step of scanning a slit defining structure across a portion of the field of said area detector.

0166567

FIG. 1

FIG. 2

C166567

FIG. 3

FIG. 4A — $t = T_1$

FIG. 4B — $t = T_2$

FIG. 4C — $t = T_3$

FIG. 4D — $t = T_4$

0166567

38

70

78

72

74

FIG. 5

80

84

82

FIG. 6

88

86

FIG. 7

RADIATION IN

96

92

98

RADIATION SENSITIVE PHOSPHOR OR SCINTILLATO

96

94

90

CHARGE COUPLED IMAGE CHIPS ARE MOUNTED ON FACES OF FIBER

FIG.8

0166567

FIG. 9

FIG. 10

0166567

CHARGE MOTION

FIG. 11

TO IMAGE PROC

O1    D1,1  D1,2                    D1,64

O2                                  150

D64  D64,1  D64,2                  D64,64

DETECTOR MOTION

FIG. 13

F AT ZERO IN

280                    270

$\phi_{1Y}$

$\phi_{2Y}$

282

64 ELEMENT BBD SHIFT REGISTER

201

(2)

263

264

TRANSFER GATE

290

286

272

F AT ZERO IN

265

276

VIDEO OUTPUT

SENSING AMPLIFIER

128 ELEMENT BBD SHIFT REGISTER

272

274  292  $\phi_{1X}$  $\phi_{2X}$  294

DETECTOR
MOTION →

58   51   D1,1   D1,2                    D1,256

TO SUMMING
AND IMAGE
PROC CKTS

170

D64,1                                  D64,256

← CHARGE MOTION

## FIG. 12

$\phi_{1Y}$   280

$\phi_{2Y}$
      282

$\phi$TRANSFER
295

      292   1   2      62   63   64   1
$\phi_{1X}$

$\phi_{2X}$
      294

## FIG. 14

0166567

SERVO

305 FROM ENCODER

300
306
304
308
302
313
312
318
314
321
322
323
320
45°
339
324
340
325
326
327

TO SERVO 305
332 334
ENC.
PIVOT APP.
330
FROM OPERATOR'S CONSOLE
326

Fig. 15

Fig. 16

0166567

Fig. 17

LIGHT    329    LIGHT

326

327

325

Fig. 18

64

1024

326

TDI CHARGE SHIFT

Fig. 19